# EUROPEAN PATENT APPLICATION

(11) **EP 4 570 284 A1**
(43) Date of publication of application: **18.06.2025**
(21) Application number: 22958459.4
(22) Date of filing: 16.09.2022
(51) Int. Cl.: A61M 5/20

(54) **MECHANICAL AUTOMATIC INJECTION PEN**

(71) Applicant: Zhejiang Summed Medtech Co., Ltd., Huzhou, Zhejiang 313000 (CN)
(72) Inventor: YE, Guohui, Jiaxing, Zhejiang 314199 (CN)
(74) Representative: Schwerbrock, Florian
(86) International application number: PCT/CN2022/119208
(87) International publication number: WO 2024/055268

(57) **Abstract**

The present invention relates to a mechanical automatic injection pen, which is used for installing a needle assembly with medicine to perform the injection operation. The mechanical automatic injection pen includes a first assembly and a second assembly that can be combined together. The first assembly includes a first pipe, a pulling assembly, a push rod, a locking assembly, a first elastic assembly, and a second elastic assembly, and the second assembly includes a second pipe and an actuating assembly. The locking assembly is actuated by the actuating assembly to release the locked state of the push rod, so that the push rod moves along the axis toward the needle assembly through the second elastic assembly to push the medicine to perform the injection operation. After the injection operation is completed, the pulling assembly can move along the axis in a direction away from the second pipe by an external force, thereby gradually driving the push rod to return to the position before the injection operation. Accordingly, the push rod can be returned to the original position by operation of the pulling assembly to achieve the effect of reusability.

## Description

### FIELD OF THE INVENTION

The present invention relates to a technical field of automatic injection pens, in particularly relates to a reusable mechanical automatic injection pen.

### BACKGROUND OF THE INVENTION

In the past, when patients needed injections of drugs, they always had to trouble medical staff to assist with the injection procedures. With the advancement of medical technology, automatic injection pens that can be operated by patients themselves have been widely used. Patients can complete the injection of drugs by operating the automatic injection pen with one hand, eliminating the consumption of medical manpower and resources, and improving the convenience and flexibility of injection.

However, most of the existing automatic injection pens are disposable injection needles, which can only be discarded after use, which easily lead to a waste of resources and money. Furthermore, in order to achieve the purpose of automatic injection, the existing automatic injection pens are structurally. The design is often too complex, resulting in increased manufacturing costs. Therefore, how to make the automatic injection pen reusable and simplify the structure and quantity of its assembled parts is a topic worthy of study.

### SUMMARY OF THE INVENTION

The present invention is made in view of the abovementioned problems, and the objective is to provide a mechanical automatic injection pen that can be used repeatedly and has a simplified structural design to reduce the waste of resources and money.

The mechanical automatic injection pen of the present invention includes a first assembly and a second assembly. The first assembly includes a first pipe, a pulling assembly, a push rod, a locking assembly, a first elastic assembly and a second elastic assembly. The first pipe includes an opposite first end and a second end; the pulling assembly is movably inserted into the first piper from a first end of the first pipe along an axis, and one end of the pulling assembly includes an opening hole; the push rod is inserted into first pipe and the pulling assembly along the axis from the second end of the first pipe, and one end of the push rod away from the needle assembly passes through the opening hole; the locking assembly is engaged with the second end of the first pipe, and locking assembly is used to lock the push rod; the two ends of the first elastic assembly respectively are in contact with the first pipe and the pulling assembly; and two ends of the second elastic member respectively are in contact with the first pipe and push rod.

The second assembly is engaged with the first assembly to form a receiving space for installing the needle assembly, and the second assembly includes a second pipe and an actuating assembly. The second pipe is engaged with a second end of the first pipe; the actuating assembly passes through the second pipe, and one end of the actuating assembly is in contact with the locking assembly. The locking assembly is actuated by the actuating assembly to release the locked state of the push rod, so that the push rod moves along the toward the needle assembly along the axis through the second elastic assembly to push the medicine to perform the injection operation; when the injection operation is completed, the pulling assembly moves along the axis toward a direction away from the second pipe by the external force, thereby gradually driving the push rod to return to the position before the injection operation, so that the push rod is locked by the locking assembly again, and the pulling assembly is returned to the position before it is moved by the external force through the first elastic assembly.

In one embodiment of the present invention, the push rod further includes a traction part. The traction part is arranged at one end of the push rod away from the needle assembly, and the cross-sectional diameter of the traction part is larger than that of the opening hole of the pulling assembly.

In one embodiment of the present invention, the locking assembly further includes at least one locking part, the push rod further includes at least one corresponding locking part. The locking part is engaged with at least one corresponding locking part of the push rod to constitute a locked state through at least one locking part.

In one embodiment of the present invention, the first pipe further includes an abutment part arranged at the first end, the pulling assembly further includes a corresponding abutment part arranged on the outer surface of the pulling assembly; two ends of the first elastic assembly respectively are in contact with the abutment part of the first pipe and the corresponding abutment part of the pulling assembly.

In one embodiment of the present invention, the actuating assembly further a first actuating end and a second actuating end. The first actuating end is in contact with the locking assembly, and the second actuating end protrudes from the second pipe and surrounds the needle of the needle assembly.

In one embodiment of the present invention, the mechanical automatic injection pen further includes a cover. The cover is combined with another end of the second assembly.

In one embodiment of the present invention, the cover further includes a needle protection part. When the cover is combined with the second assembly, the needle protection part is inserted into the second actuating end of the actuating assembly to accommodate the needle of the needle assembly.

In one embodiment of the present invention, before the mechanical automatic injection pen performs the injection operation, the first elastic assembly is in a relaxed state, and the second elastic assembly is in a compressed state.

In one embodiment of the present invention, there is at least one corresponding locking part arranged between the two ends of the push rod.

In one embodiment of the present invention, the push rod further a rubber plug, which is arranged at one end of the push rod close to the needle assembly.

The mechanical automatic injection pen of the present invention can operate the pulling assembly to prompt the push rod to return to its position after the injection operation is completed, so that the user can replace the new needle assembly, thereby achieving the reusable effect of the mechanical automatic injection pen to avoid wastage of the resource and money. In addition, the mechanical automatic injection pen of the present invention can effectively simplify the structural design of related components and improve the convenience of assembly.

The specific technology used in the present invention will be further explained through the following examples and attached drawings.

### BRIEF DESCRIPTION OF THE DRAWINGS

Fig. 1 is a schematic diagram of the appearance of the mechanical automatic injection pen of the present invention.
Fig. 2 is an exploded view of the mechanical automatic injection pen of the present invention.
Fig. 3 is a cross-sectional view of the mechanical automatic injection pen of the present invention.
Fig. 4 is a cross-sectional view of the mechanical automatic injection pen of the present invention after performing injection operation.
Fig. 5 is a cross-sectional view of the mechanical automatic injection pen of the present invention that returns the push rod to the position before the injection operation after injection operation is completed.

Description of main component symbols:
1 mechanical automatic injection pen
10 first assembly
11 first pipe
111 first end
112 second end
113 abutment part
12 pulling assembly
121 opening hole
122 corresponding abutment part
13 push rod
131 traction part
132 corresponding locking part
134 rubber plug
14 locking assembly
141 elastic arm
142 locking part
15 first elastic assembly
16 second elastic assembly
20 second assembly
21 second pipe
211 inspection hole
22 actuating assembly
221 first actuating end
222 second actuating end
30 cover
31 needle protection part
90 needle assembly
91 needle
L axis

### DETAILED DESCRIPTION OF THE PREFERRED EMBODIMENT

Since various aspects and embodiments are only illustrative and non-limiting, after reading this specification, a person with ordinary knowledge may also have other aspects and embodiments without departing from the scope of the present invention. According to the following detailed description and patent application scope, the features and advantages of these embodiments will be more clearly demonstrated.

In this application, "a" or "an" is used to describe the components and components described herein. This is done only for the convenience of explanation and to provide a general sense of the scope of the invention. Therefore, unless it is obvious otherwise, such description should be understood to include one or at least one, and the singular also includes the plural.

In this application, the terms "first" or "second" and similar ordinal numbers are mainly used to distinguish or refer to the same or similar components or structures, and do not necessarily imply the spatial or temporal order of these components or structures. It should be understood that in certain situations or configurations, the ordinal number can be used interchangeably without affecting the implementation of the present invention.

In this application, the terms "include", "have" or any other similar terms are intended to cover a non-exclusive inclusion. For example, a component or structure comprising multiple components is not limited to only those components listed herein but may include other components not expressly listed but that are generally inherent to the component or structure.

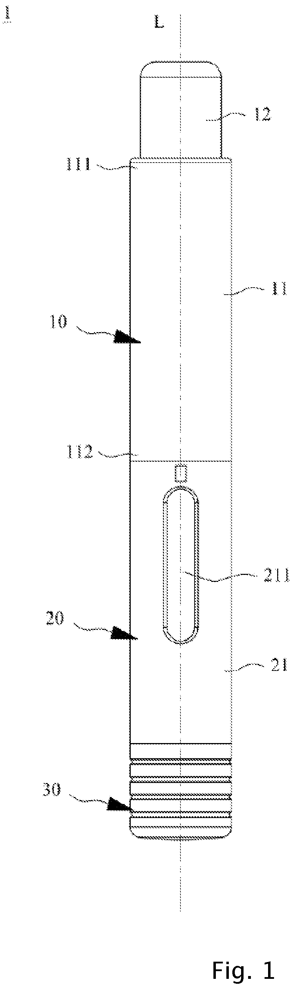

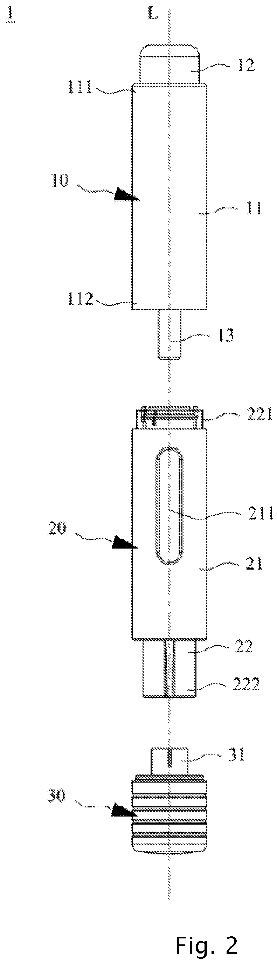

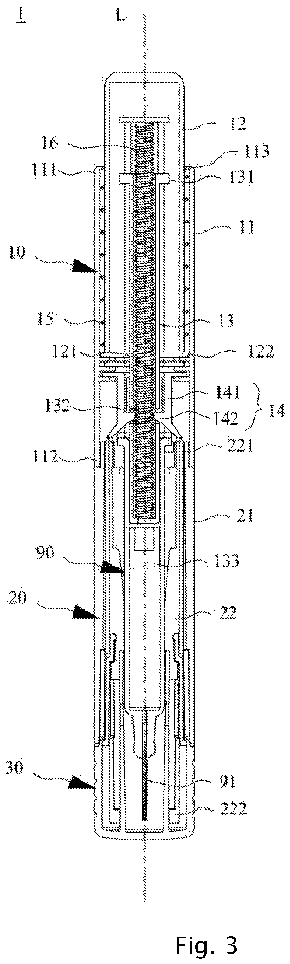

The mechanical automatic injection pen of the present invention can be equipped with the needle assembly with medicine, and after being actuated, a push rod pushes the medicine to perform the injection operation on the patient, and a new needle assembly can be replaced after injection operation is completed to achieve the effect of repeated use. Please refer to Fig. 1 to Fig. 3, in which Fig. 1 is a schematic diagram of the appearance of the mechanical automatic injection pen of the present invention, Fig. 2 is an exploded view of the mechanical automatic injection pen of the present invention, and Fig. 3 is a cross-sectional view of the mechanical automatic injection pen of the present invention.

As shown in Fig. 1 to Fig. 3, the mechanical automatic injection pen of the present invention includes a first assembly 10 and a second assembly 20, and the first assembly 10 and the second assembly 20 are combined to correspond to the axis L. Therefore, each movable part of the first assembly 10 and/or the second assembly 20 can move along the direction of axis L relative to other parts. There is a receiving space that can be formed in the second assembly 20 which is used for installing needle assembly 90 therein. Therefore, when the first assembly 10 and the second assembly 20 are separated, the user can arrange the needle assembly 90 in the second assembly 20 or replace the needle assembly 90. Each component in the first assembly 10 and the second assembly 20 can be made of the same or different solid materials, such as plastic materials, metals or alloys, but it is not limited thereto.

The first assembly 10 includes a first pipe 11, a pulling assembly 12, a push rod 13, a locking assembly 14, a first elastic assembly 15, and a second elastic assembly 16. In terms of structural design, the first pipe 11, the pulling assembly 12, the push rod 13, and the locking assembly 14 are all similar to the tube in appearance, so that the hollow parts of the aforementioned components can accommodate or pass through the corresponding components, but the present invention is not limited herein.

The first pipe 11 includes an opposite first end 111 and a second end 112, and both the first end 111 and the second end 112 are open end. In one embodiment of the present invention, the first pipe 11 further includes an abutment part 113, which is provided at the first end 111. For example, the abutment part 113 is a flange structure extending inward along the vertical axis L direction, but the present invention is not limited herein.

The pulling assembly 12 is movably inserted into the first pipe 11 from the first end of the first pipe 11 along the axis L, and pulling assembly 12 can move along the axis L relative to the first pipe 11. One end of the pulling assembly 12 inserted into the first pipe 11 includes an opening hole 121, so that this end constitutes an open end, while the other opposite end of the pulling assembly 12 is a closed end. In one embodiment of the present invention, the pulling assembly 12 further includes a corresponding abutment part 122, which is disposed on outer surface of the pulling assembly 12, for example, the corresponding abutment part 122 is disposed close to the aforementioned open end.

In order to cooperate with the abutment part 113 of the first pipe 11, the corresponding abutment part 122 can be a flange extending outward along the vertical axis L direction, but the present invention is not limited herein. The abutment part 113 of the first pipe 11 cooperates with the corresponding abutment part 122 of the pulling assembly 12, so that the pulling assembly 12 conducts stop effect while the pulling assembly 12 moves relative to the first pipe 11 along the axis L and does not separate from the first pipe 11.

The push rod 13 inserted into the first pipe 11 and the pulling assembly 12 from the second end 112 of the first pipe 11 along the axis L, and the push rod 13 can move relative to the first pipe 11 and the pulling assembly 12 along the axis L. One end of the push rod 13 is disposed away from the needle assembly 90 passes through the opening hole 121 of the pulling assembly 12. In one embodiment of the present invention, the push rod 13 further includes a traction part 131, which is disposed at one end of the push rod 13 away from the needle assembly 90, so the traction part 131 is disposed between the open end of the pulling assembly 12 having an opening hole 121 and the opposite closed end. The traction part 13 is arranged on an outer surface of the push rod 13, for example, the traction part 13 is a flange extending outward in the direction of the vertical axis L, but the invention is not limited herein.

In terms of structural design, the cross-sectional diameter of traction part 131 is larger than that of the opening hole 121 of the pulling assembly 12. That is to say, during the movement of the push rod 13, the traction part 131 cannot pass through the opening hole 121 of the pulling assembly 12, the push rod 13 not only conducts the stop effect when the push rod 13 moves along axis L relative the first pipe 11 and the pulling assembly 12, but it will not separate from the pulling assembly 12. The push rod 13 can also drive by the traction part 131 to move through the pulling assembly 12.

The push rod 13 further includes at least one corresponding locking part 132. The corresponding locking part 132 is disposed between both ends of the push rod 13. In this embodiment, the push rod 13 can include two corresponding locking parts 132 arranged symmetrically, each corresponding locking part 132 is a groove structure, but the structure and the number of corresponding locking part 132 are not limited herein. In one embodiment of the present invention, the push rod 13 further includes a rubber plug 133. The rubber plug 133 is arranged at one end of the push rod 13 close to the needle assembly 90, that is, the one end opposite to the end of the push rod 13 includes traction part 131.

The locking assembly 14 is engaged with the second end of the first pipe 11. The locking assembly 14 is used to lock the push rod 13 before performing the injection operation or after the completing the injection operation. In one embodiment of the present invention, the locking assembly 14 includes at least one locking part 142, and the locking assembly 14 is engaged with at least one corresponding locking part 132 of the push rod 13 to constitute a locked state through at least one locking part 142. In this embodiment, locking assembly 14 can include two symmetrically arranged locking parts 142, and each locking part 142 is a convex structure matching with the aforementioned groove structure. Each locking part 142 can arrange on the corresponding elastic arm 141, but the structure and the number of the locking part 142 are not limited herein.

The first elastic assembly 14 is disposed between the first pipe 11 and the pulling assembly 12, and the two ends of the first elastic assembly 15 are respectively in contact with the first pipe 11 and the pulling assembly 12, so that the pulling assembly 12 can move relative to the first pipe 11 to return to the original position through the first elastic assembly 15. In one embodiment of the present invention, both ends of the first elastic assembly 15 are in contact with the abutment part 113 of the first pipe 11 and the corresponding abutment part 122 of the pulling assembly 12. In this present invention, the first elastic assembly 15 is in a relaxed state before performing injection operation.

The second elastic assembly 16 is disposed between the first pipe 11 and the push rod 13, and both ends of the second elastic assembly 16 are respectively in contact with the first pipe 11 and the push rod 13, so the push rod 13 can move toward the needle assembly 90 by the elastic driving force provided by the second elastic assembly 16. In the present invention, the second elastic assembly 16 is in a compressed state before performing injection operation.

The second assembly 20 can be combined with the first assembly t 10 and the cover 30. The second assembly 20 includes a second pipe 21 and an actuating assembly 22. In terms of structural design, the second pipe 21 and the actuating assembly 22 are similar to the tube in appearance, so that the hollow parts of the aforementioned components can accommodate the corresponding components, but the invention is not limited herein.

The second pipe 21 includes two opposite ends, and both ends are open end. One end of the second pipe 21 is combined with the second end 112 of the first pipe 11.

The actuating assembly 22 is inserted through the second pipe 21, and one end of the actuating assembly 22 is in contact with the locking assembly 14. In one embodiment of the present invention, the actuating assembly 22 includes a first actuating end 221 and a second actuating end 222. The actuating assembly 22 is in contact with the locking assembly 14 through the first actuating end 221, and the second actuating end 222 protrudes from the second pipe 21 and surrounds the needle 91 of the needle assembly 90. Accordingly, the actuating assembly 22 can provide the protective effect for the needle 91 of the needle assembly 90 through the second actuating end 222 before performing the injection operation, and the locking assembly 14 is actuated by the first actuating end 221 during the performing injection operation.

In one embodiment of the present invention, the mechanical automatic injection pen 1 further includes a cover 30, and the cover 30 is combined with another end of the second assembly 20 to shield and protect the needle assembly 90. In one embodiment of the present invention, the cover 30 includes a needle protection part 31. When the cover 30 is combined with the second assembly 20, the needle protection part 31 is inserted into the second actuating end 222 of the actuating assembly 22 and is to accommodate the needle 91 of the needle assembly 90.

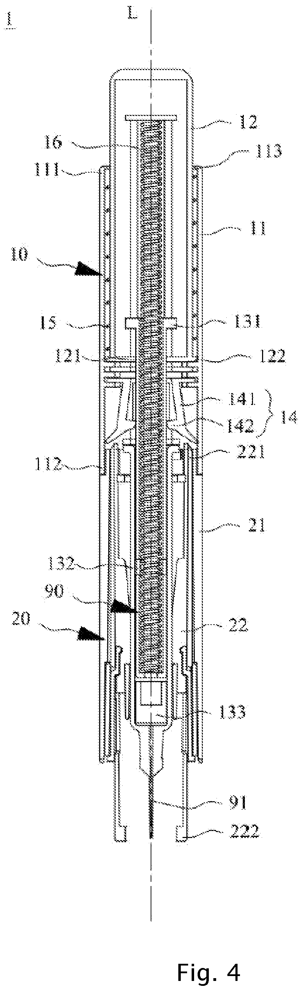

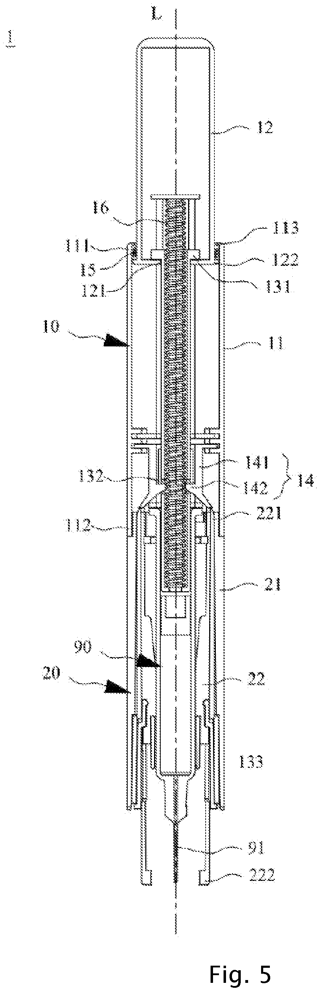

The following will further illustrate the corresponding structural movements of the mechanical automatic injection pen 1 of the present invention before and after the injection operation is performed with reference to Fig. 3 to Fig. 5, in which Fig. 4 is a cross-sectional view of the mechanical automatic injection pen of the present invention after performing injection operation, and Fig. 5 is a cross-sectional view of the mechanical automatic injection pen of the present invention returns the push rod to original position before the injection operation after injection operation is completed.

As shown in Fig. 3, when the user wants to use the mechanical automatic injection pen 1 of the present invention, the cover 30 is taken off first, so the second actuating end 222 of the actuating assembly 22 is exposed. Next, the user can hold the mechanical automatic injection pen 1, the exposed second actuating end 222 of the actuating assembly 22 is in contact with the position of the human body that is to be injected and apply force toward the skin of the human body, so the actuating assembly 22 is moved toward the first assembly 10. at this time, the first actuating end 221 of the actuating assembly 22 will push against the locking assembly 14 of the first assembly 10, so each the elastic arms 141 of the locking assembly 14 drives the corresponding locking part 142 to move in a direction toward away from the axis L and separate from the corresponding locking part 132 of the push rod 13, thereby the locking assembly 14 releases the locked state of the push rod 13.

Accordingly, the push rod 13 is actuated by the compressed second elastic assembly 16 to move toward the needle assembly 90 along axis L without interference, and then rubber plug 133 of the push rod 13 pushes the medicine in the needle assembly 90 and makes the medicine discharged through the needle 91 to perform the injection operation. When the injection operation is completed, the mechanical automatic injection pen 1 of the present invention will be in state as shown in Fig. 4.

As shown in Fig. 4, when the injection operation is completed, the user can first move the mechanical automatic injection pen 1 of the present invention from the human body. Then, the user can apply external force to the pulling assembly 12, so that the pulling assembly 12 moves relative to the first pipe 11 along the axis L in a direction away from the second pipe 21. During the movement of the pulling assembly 12, the open end of the pulling assembly 12 having an opening hole 121 will contact the traction part 131 of the push rod 13, since the cross-sectional diameter of the traction part 131 of the push rod 13 is larger than that of the opening hole 121 of the pulling assembly 12, so that the pulling assembly 12 can push against the traction part 131 of the push rod 13 through the open end to drive the push rod 13 move toward in a direction away from the direction of the needle assembly 90.

As the push rod 13 gradually moves and returns to the original position before the injection operation, at least one corresponding locking part 132 of the push rod 13 will engage with at least one locking part 142 of the locking assembly 14 to constitute a locked state, so the push rod 13 is locked by the locking assembly 14 again. In addition, as the pulling assembly 12 is moved relative to the first pipe 11, the first elastic assembly 13 will be compressed to accumulate the elastic potential energy.

In terms of the structural design, since each locking part 142 of the locking assembly 14 will engage with each corresponding locking part 132 of the push rod 13 through the corresponding elastic arm 141, so as to generate the elastic force to impact to generate the sound, so the user can according to the sound to confirm whether the push rod 13 has been locked. After the user confirms that push rod 13 has been locked, the user can stop applying external force to the pulling assembly 12. At this time, the pulling assembly 12 moves relative to the opposite direction of the first pipe 11 to return to the original position before receiving the external force. Finally, the mechanical automatic injection pen 1 will return to the state before performing the injection operation as shown in Fig. 1, so that the user can replace the needle assembly 90 with new one and reuse it repeatedly.

In addition, in terms of the structural design, the second pipe 11 further includes an inspection part 211. The inspection part 211 allows the user to confirm the injection state of the medicine inside the needle assembly 90. The inspection part 211 is arranged on the outer surface of the second pipe 21. In one embodiment of the present invention, the inspection part 221 can be made of a transparent material to facilitate the user to inspect the needle assembly 90 through the inspection part 221.

The above embodiments are essentially only for auxiliary explanation and are not intended to limit the application or use of the embodiment or multiple embodiments of the application target. Furthermore, although at least one exemplary embodiment has been presented in the foregoing detailed description, it should be appreciated that a vast number of variations are possible in the present invention. It should also be appreciated that the embodiments described herein are not intended to limit the scope, applicability, or configuration of the claimed subject matter in any way. On the contrary, the foregoing detailed description will provide a person skilled in the art with a convenient guide for implementing one or more of the described embodiments. Furthermore, various changes can be made in the function and arrangement of elements without departing from the scope defined by the claims, and the claims include known equivalents and all foreseeable equivalents at the time of filing this patent application.

## Claims

1. A mechanical automatic injection pen is used to install a needle assembly with a medicine to perform an injection operation, **characterized in that**, the mechanical automatic injection pen comprising:
a first assembly, comprises:
a first pipe includes an opposite first end and a second end;
a pulling assembly, which is movable inserted into the first pipe along an axis from the first end of the first pipe, and one end of the pulling assembly includes an opening hole;
a push rod, which is inserted into the first pipe and the pulling assembly from the second end of the first pipe along the axis, and one end of the push rod away from the needle assembly through the opening hole;
a locking assembly, coupled to the second end of the first pipe, the locking assembly is used to lock the push rod;
a first elastic assembly, the two ends of the first elastic assembly are in contact with the first pipe and the pulling assembly respectively; and
a second elastic assembly, the two ends of the second elastic assembly are in contact with the first pipe and the push rod; and
a second assembly, combined with the first assembly along the axis to form a receiving space for installing the needle assembly, the second assembly comprises:
a second pipe, coupled to the second end of the first pipe; and
an actuating assembly, inserted through the second pipe, and one end of the actuating assembly is in contact with the locking assembly;
wherein, the locking assembly is actuated by the actuating assembly to release a locked state of the push rod, so that the push rod moves toward the needle assembly along the axis through the second elastic assembly to push the medicine to perform an injection operation; when the injection operation is completed, the pulling assembly moves along the axis toward a direction away from the second pipe by an external force, thereby gradually driving the push rod to return to the position before the injection operation, so that the push rod is locked by the locking assembly again, and the pulling assembly is returned to the position before it is moved by the external force through the first elastic assembly.

2. The mechanical automatic injection pen according to claim 1, the **characterized in that**, wherein the push rod further comprises a traction part, the traction part is arranged at one end of the push rod away from the needle assembly, and the cross-sectional diameter of the traction part is larger than that of the opening hole of the pulling assembly.

3. The mechanical automatic injection pen according to claim 2, the **characterized in that**, wherein the locking assembly further comprises at least one locking part, the push rod further comprises at least one corresponding locking part, and the locking assembly is engaged with at least one corresponding locking part of the push rod through at least one locking part to constitute a locked state.

4. The mechanical automatic injection pen according to claim 1, the **characterized in that**, wherein the first pipe further comprises an abutment part arranged at the first end, the pulling assembly further comprises a corresponding abutment part arranged on an outer surface of the pulling assembly, two ends of the first elastic assembly respectively are in contact with the abutment part of the first pipe and the corresponding abutment part of the pulling assembly.

5. The mechanical automatic injection pen according to claim 1, the **characterized in that**, wherein the actuating assembly further comprises a first actuating end and a second actuating end, the first actuating end is in contact with the locking assembly, the second actuating end protrudes from the second pipe and surrounds a needle of the needle assembly.

6. The mechanical automatic injection pen according to claim 5, the **characterized in that**, wherein further comprising a cover, the cover being combined with another end of the second assembly.

7. The mechanical automatic injection pen according to claim 6, the **characterized in that**, wherein the cover further comprising a needle protection part, when the cover is combined with the second assembly, the needle protection part is used to insert into the second actuating end of the actuating assembly to accommodate the needle of the needle assembly.

8. The mechanical automatic injection pen according to claim 1, the **characterized in that**, wherein before the mechanical automatic injection pen performs the injection operation, the first elastic assembly is in a relaxed state, and the second elastic assembly is in a compressed state.

9. The mechanical automatic injection pen according to claim 1, the **characterized in that**, wherein at least one corresponding locking assembly arranged between the two ends of the push rod.

10. The mechanical automatic injection pen according to claim 1, the **characterized in that**, wherein the push rod further comprises a rubber plug arranged at one end of the push rod close to the needle assembly.
